# EUROPEAN PATENT APPLICATION

(11) **EP 2 275 437 A1**
(43) Date of publication of application: **19.01.2011**
(21) Application number: 09722434.9
(22) Date of filing: 17.03.2009
(51) Int. Cl.: C07K 14/47, A61K 38/00, A61P 9/00, A61P 9/10, A61P 17/00, A61P 17/06, A61P 19/02, A61P 27/02, A61P 29/00, A61P 35/00, C12N 15/00

(54) **POLYPEPTIDE AND PHARMACEUTICAL COMPOSITION CONTAINING THE POLYPEPTIDE**

(30) Priority: 18.03.2008 JP 2008070434
(71) Applicant: National University Corporation Hokkaido University, Sapporo-shi Hokkaido 060-0808 (JP); Kurume University, Kurume-shi Fukuoka 8300011 (JP)
(72) Inventor: NAKAMURA, Kazuo, Kurume-shi Fukuoka 830-0011 (JP); MATSUI, Takanori, Kurume-shi Fukuoka 830-0011 (JP); IMAIZUMI, Tsutomu, Kurume-shi Fukuoka 830-0011 (JP); YAMAGISHI, Sho-ichi, Kurume-shi Fukuoka 830-0011 (JP); ABE, Riichiro, Sapporo-shi Hokkaido 060-8648 (JP); SHIMIZU, Hiroshi, Sapporo-shi Hoikkaido 060-8638 (JP)
(74) Representative: Oudin, Stéphane
(86) International application number: PCT/JP2009/055165
(87) International publication number: WO 2009/116529

(57) **Abstract**

Disclosed is a novel polypeptide which can inhibit angiogenesis. Also disclosed is use of the polypeptide. The polypeptide comprises at least one amino acid sequence selected from the amino acid sequences depicted in SEQ ID NOs: 1-3, is composed of 120 or less animo acid residues, and has an anti-angiogenic activity. The polypeptide has an anti-antiogenic activity, has a lower molecular weight compared with that of PEDF, and has excellent penetration or absorption into a living body, particularly an affected part in a living body.

## Description

### Technical field

The present invention relates to a polypeptide effective for treating or preventing diseases involving angiogenesis, an anti-angiogenic agent having the polypeptide as an active ingredient, or a pharmaceutical composition containing the polypeptide.

### Background Art

In a human body, angiogenesis is induced only at an appropriate time or in an appropriate site, for example, in wound healing and a specific stage of menstrual period, under strict regulation by a functional balance between an angiogenesis-promoting factor and an anti-angiogenic factor.

However, it is known that, in some diseases, the above regulation is disabled for some reasons, causing induction of undesirable angiogenesis and exacerbation of symptoms. For example, in diabetic retinopathy and age-related macular degeneration, neovascularization in the iris, the retina, or the optic nerve has been observed, which is known to cause serious vision loss. Further, it is known that angiogenesis is induced also in a number of solid tumors, and while the newly-formed blood vessel supplies blood to tumor cells to grow the tumor and cause damage to surrounding normal tissues, it promotes metastasis of the tumor cells to other sites or tissues via blood stream.

Some of the factors associated with regulation of angiogenesis have been already identified. Representative examples of angiogenesis-promoting factor include a vascular endothelial growth factor (VEGF), an α-subunit of hypoxia-inducible factor 1 (HIF-1), a 110 kDa member of immunoglobulin superfamily, angiopoietin-1 (Ang1), and angiopoietin-2 (Ang2). Further, Flt-1 and FLk-1/KDR receptors, which are kinds of membrane-spanning protein tyrosine kinase expressed on the surface of endothelial cells, also initiate, by binding to VEGF, a cell signal cascade causing the final angiogenesis in surrounding tissues. Thus, they are also understood to be angiogenesis-promoting factors.

Meanwhile, representative examples of anti-angiogenic factor include a pigment epithelium-derived factor (PEDF), endostatin, and TIMP-3. It is reported that these anti-angiogenic factors inhibit angiogenesis and exert certain therapeutic effects when administered into a site where angiogenesis has been induced. For example, in a model animal administered with adeno-associated viral vectors expressing PEDF, inhibition of capillary angiogenesis in retinal epithelial cells has been confirmed (Patent Document 1). Further, it has been known that angiogenesis is inhibited also in a model animal administered with adeno-associated viral vectors expressing angiostatin and that retinal angiogenesis is inhibited also in a mouse model of retinopathy of prematurity by subretinal injection of the endostatin (Non-Patent Document 2).

Among these anti-angiogenic factors, application of PEDF (Non-Patent Document 1) as an anti-angiogenic agent has been attracting attention for its strong anti-angiogenic action (for example, Patent Document 1 and Patent document 3).

Also, Patent Document 4 has proposed use of PEDF or a polypeptide having 44 amino acid residues comprising the amino acid sequence of positions 78 to 121 of PEDF (PEDF44AA peptide) in the treatment of diseases associated with hyperangiogenesis or vascular hyperpermeability. Patent Document 1 has concluded that all of the physiological functions known to be exerted by PEDF are present in the region of the aforementioned 44 amino acids, and among them, specific amino acid residues are particularly essential for functional expression of PEDF and the PEDF44AA peptide.

Non-Patent Document 1: Steele et al., 1993, Proc. Natl. Acad. USA, Vol. 90, No. 4, pp. 1526-1530
Patent Document 1: National Publication of International Patent Application 2007-528903
Patent Document 2: National Publication of International Patent Application 2004-517117
Patent Document 3: National Publication of International Patent Application: 2004-516001
Patent Document 4: National Publication of International Patent Application 2007-509984

### Disclosure of the Invention

### Problems to be Solved by the Invention

An object of the present invention is to provide a novel polypeptide capable of inhibiting angiogenesis and use of the polypeptide.

### Means for Solving the Problem

The present inventors searched for a novel polypeptide having an anti-angiogenic activity that can be an alternative to PEDF. As a result, they have surprisingly found that a polypeptide having a specific amino acid sequence present near the C-terminus of PEDF, which is completely different from the region of the amino acids of positions 78 to 121 of PEDF, exhibits an anti-angiogenic effect, thereby completing each of the below-described inventions. That is, the present invention is as follows.

(1) A polypeptide having an anti-angiogenic action, comprising any one or more of amino acid sequences shown in SEQ ID NOs: 1 to 3 and having 120 or less amino acid residues.

(2) The polypeptide according to (1), comprising the amino acid sequence shown in SEQ ID NO: 4 (F3).

(3) The polypeptide according to (1), having 20 or less amino acid residues.

(4) The polypeptide according to (3), comprising the amino acid sequence shown in SEQ ID NO: 5 (P5).

(5) The polypeptide according to (1), comprising any of the amino acid sequences shown in SEQ ID NOs: 1 to 3 (P5-1 to 3).

(6) A therapeutic agent for psoriasis comprising a polypeptide comprising the amino acid sequence shown in SEQ ID NO: 6 and having an anti-angiogenic action (PEDF) as an active ingredient.

(7) An anti-angiogenic agent comprising the polypeptide according to any of (1) to (5) as an active ingredient.

(8) A therapeutic agent for psoriasis comprising the polypeptide according to any of (1) to (5) as an active ingredient.

(9) A tumor cell proliferation-inhibiting agent comprising the polypeptide according to any of (1) to (5) as an active ingredient.

(10) An anti-angiogenic pharmaceutical composition comprising the polypeptide according to any of (1) to (5) and a pharmaceutically acceptable carrier or excipient.

(11) A pharmaceutical composition for treating psoriasis comprising the polypeptide according to any of (1) to (5) and a pharmaceutically acceptable carrier or excipient.

(12) The pharmaceutical composition according to (10) or (11), being provided as an external agent.

### Advantages of the Invention

The polypeptide of the present invention is a lower molecular polypeptide compared to PEDF and retains an anti-angiogenic action. It can overcome a living body's barrier mechanism against invasion of foreign substances such as a skin barrier, and has a characteristic that it has an excellent penetration or absorption into a living body, particularly into an affected part. Also, since a lower molecular peptide can be produced by chemical synthesis, it can be produced more cheaply at a larger-scale compared to PEDF, the production of which requires recombinant technology, leading to a considerable reduction in the production cost of medicines. Further, as a low molecular polypeptide generally has more excellent stability compared to a macromolecule such as protein, the polypeptide is advantageous to be utilized as a medicine, which is to be prepared in various forms.

### Brief Description of the Drawings

Figure 1 is a schematic diagram showing arrangement of each polypeptide with respect to PEDF;
Figure 2 is a graph showing the inhibitory effects of PEDF and polypeptides F1 to F3 on proliferation of MG63, human osteosarcoma cells. In the figure, Con indicates a control to which PBS was added;
Figure 3 is a graph showing the inhibitory effects of polypeptides P1 to P6 on proliferation of MG63, human osteosarcoma cells. In the figure, Con indicates a control to which PBS was added;
Figure 4 is a graph showing the inhibitory effects of polypeptides P5-1 to P5-3 on proliferation of MG63, human osteosarcoma cells. In the figure, Con indicates a control to which PBS was added;
Figure 5 is a graph showing the inhibitory effects of polypeptides P5-1 to P5-3 on proliferation of HUVECs. In the figure, Con indicates a control to which PBS was added;
Figure 6 is a graph showing changes in the thickness of the psoriasis patient-derived skin grafted onto the back of a mouse. In the figure, control peptide indicates a control to which a peptide having a random sequence was added;
Figure 7 is a graph showing changes in the number of capillary endothelial cells in the psoriasis patient-derived skin grafted onto the back of a mouse. In the figure, control peptide indicates a control to which a peptide having a random sequence was added;
Figure 8 shows photographs exhibiting the thickness of the psoriasis patient-derived skin grafted onto the back of mice each receiving subcutaneous injection of either PEDF protein or PBS;
Figure 9 is a graph showing the thickness of the psoriasis patient-derived skin grafted onto the back of mice each receiving subcutaneous injection of either PEDF protein or PBS;
Figure 10 shows photographs exhibiting the thickness of the normal individual-derived skin grafted onto the back of mice each receiving subcutaneous injection of either PEDF protein or PBS;
Figure 11 is a graph showing the thickness of the normal individual-derived skin grafted onto the back of mice each receiving subcutaneous injection of either PEDF protein or PBS;
Figure 12 is a graph showing the number of CD31-positive capillary endothelial cells in the psoriasis patient-derived skin grafted onto the back of mice each receiving subcutaneous injection of either PEDF protein or PBS;
Figure 13 is a graph showing the number of CD31-positive capillary endothelial cells in the normal individual-derived skin grafted onto the back of mice each receiving subcutaneous injection of either PEDF protein or PBS;
Figure 14 shows the frequency of Ki67-positive cells in the basal cell layer of the psoriasis patient-derived skin grafted onto the back of a mouse; and
Figure 15 shows the frequency of Ki67-positive cells in the basal cell layer of the normal individual-derived skin grafted onto the back of a mouse.

### Best Mode for Carrying Out the Invention

The present invention provides a polypeptide having an anti-angiogenic action, which comprises any one or more of amino acid sequences shown in SEQ ID NOs: 1 to 3 and having 120 or less amino acid residues.

In a preferred aspect, the aforementioned polypeptide is a polypeptide comprising the amino acid sequence shown in SEQ ID NO: 4. Hereinafter, such a polypeptide is expressed as F3. The amino acid sequence of F3 is identical to the amino acid sequence of positions 301 to 418 of PEDF (the entirety of the amino acid sequence thereof is shown in SEQ ID NO: 6).

In another preferred aspect, the aforementioned polypeptide is a polypeptide having an anti-angiogenic action, which comprises any one or more of amino acid sequences shown in SEQ ID NOs: 1 to 3 and has 20 or less amino acid residues. More preferably, it is a polypeptide comprising the amino acid sequence shown in SEQ ID NO: 5. Hereinafter, such a polypeptide is expressed as P5. The amino acid sequence of P5 is identical to the amino acid sequence of positions 381 to 400 of PEDF.

In a most preferred aspect, the aforementioned polypeptide is a polypeptide having an anti-angiogenic action, which comprises any one or more of amino acid sequences shown in SEQ ID NOs: 1 to 3. The amino acid sequences shown in SEQ ID NOs: 1 to 3 are each identical to the amino acid sequences of positions 381 to 387, 388 to 393, and 394 to 400 of PEDF, respectively. The polypeptides comprising the amino acid sequences shown in SEQ ID NOs: 1 to 3 are each expressed as P5-1, P5-2, and P5-3, respectively.

As shown in Examples to be described later, the polypeptide of the present invention has an inhibitory activity on growth of MG63 cell, which is a human osteosarcoma cell, or normal human umbilical vein endothelial cells (HUVECs), similarly to PEDF. This means that the polypeptide of the present invention has an anti-angiogenic action, similarly to PEDF. Accordingly, the polypeptide of the present invention can be utilized as an anti-angiogenic agent. It is to be noted that, in the present invention, "inhibiting angiogenesis" and "an anti-angiogenic agent" can be used interchangeably with "blocking angiogenesis" and "an angiogenesis-blocking agent." Accordingly, "an anti-angiogenic action" can be expressed also as "an angiogenesis-blocking action", and "an anti-angiogenic agent" can be expressed also as "an angiogenesis-blocking agent."

As described above, Patent Document 4 describes that the segment governing the function of PEDF is the segment of the amino acid sequence of positions 78 to 121 located in the N-terminal side of PEDF. The amino acid sequence of positions 78 to 121 is completely different from the amino acid sequence constituting the polypeptide of the present invention, and these amino acid sequences do not overlap at all either on PEDF. It is rare that functional protein such as PEDF has multiple so-called "active centers" independently and separately in different positions within single protein molecule. Thus, the present invention provides a previously-unknown, totally new finding pertaining to the structure-function relationship of PEDF.

It is to be noted that F3 is one example of "a polypeptide having an anti-angiogenic action, comprising any one or more of amino acid sequences shown in SEQ ID NOs: 1 to 3 and having 120 or less amino acid residues." With respect to the amino acid sequence of F3, an amino acid sequence in which one or several amino acids other than the amino acids constituting the amino acid sequence corresponding to any of SEQ ID NOs: 1 to 3 is substituted or deleted or a polypeptide comprising an amino acid sequence obtained by addition of amino acid(s) to the amino acid sequence of F3 within a range that the number of amino acid residues does not exceed 120 is encompassed by the present invention as long as it retains an anti-angiogenic effect.

Also, P5 is one example of "a polypeptide having an anti-angiogenic action, comprising any one or more of amino acid sequences shown in SEQ ID NOs: 1 to 3 and having 20 or less amino acid residues." With respect to the amino acid sequence of P5, an amino acid sequence in which one or several amino acids other than the amino acids constituting the amino acid sequence corresponding to any of SEQ ID NOs: 1 to 3 is substituted or deleted or a polypeptide comprising an amino acid sequence obtained by addition of amino acid(s) to the amino acid sequence of P5 within a range that the number of amino acid residues does not exceed 120 is encompassed by the present invention as long as it retains an anti-angiogenic effect.

"One or several" pertaining to substitution and deletion of the amino acid sequence of F3 means within one to several tens, preferably within one to 30, more preferably within one to 20, even more preferably within one to 10, and most preferably within one to five. Also, in terms of identity (%) of the amino acid sequence based on the amino acid sequence of F3, it can be expressed as an amino acid sequence having an identity of 80% or higher, preferably 85% or higher, more preferably 90% or higher, and particularly preferably 95% or higher with respect to each of the amino acid sequences. Also, "one or several" pertaining to substitution and deletion of the amino acid sequence of P5 means within one to 10, preferably within one to five, and more preferably within one to three. Also, in terms of identity (%) of the amino acid sequence based on the amino acid sequence of P5, it can be expressed as an amino acid sequence having an identity of 80% or higher, preferably 85% or higher, more preferably 90% or higher, and particularly preferably 95% or higher with respect to each of the amino acid sequences.

It has been empirically known that, in an amino acid sequence of protein, a highly conservative mutation in physicochemical properties of an amino acid residue such as electric charge, size, and hydrophobicity can be tolerated. Examples of substitution of amino acid residues include glycine (Gly) and proline (Pro), Gly and alanine (Ala) or valine (Val), leucine (Leu) and isoleucine (Ile), glutamic acid (Glu) and glutamine (Gln), aspartic acid (Asp) and asparagine (Asn), cysteine (Cys) and threonine (Thr), Thr and serine (Ser) or Ala, lysine (Lys) and arginine (Arg). Further, even when a mutation goes beyond the aforementioned tolerable conservation, those skilled in the art have empirically experienced that the mutation that would not extinguish the essential function of the protein could exist. Accordingly, also in the polypeptide of the present invention, there are cases that a polypeptide comprising an amino acid obtained by substitution, deletion, and/or addition of one or several amino acids still retains an anti-angiogenic action. Such a polypeptide is still understood to be one aspect of the present invention.

Also, the polypeptide of the present invention can be produced or used as a so-called fusion polypeptide, which is obtainable by adding a polypeptide having a different function from that of the polypeptide of the present invention to the N-terminus and/or the C-terminus of the polypeptide of the present invention. Such a fusion polypeptide is also one aspect of the present invention. Considering that the function of the added polypeptide is additionally imparted, there might be a case that the fusion polypeptide has enhanced utility compared to the case that the polypeptide of the present invention is produced or used alone. Examples of such a functional polypeptide include glutathione-S-transferase, fluorescent protein, a FLAG tag, a histidine tag, and a chitin-binding sequence.

Further, it is possible to add an appropriate labeling compound such as a fluorescent substance and a radioactive substance and connect various chemically-modifying substances and a polymer such as polyethylene glycol to the polypeptide of the present invention as needed. Alternatively, it is also possible to connect the polypeptide of the present invention to an insoluble carrier. Chemical modification methods targeting a polypeptide are widely known among those skilled in the art, and the polypeptide used in the present invention can be modified and utilized in any way as long as the function thereof is not impaired. It is to be noted that because the polypeptide of the present invention exhibits an anti-angiogenic action in the unglycosylated form, it is not necessarily required to perform glycosylation of the polypeptide of the present invention.

The anti-angiogenic action of the polypeptide of the present invention can be confirmed by, for example, methods known by those skilled in the art such as an angiogenesis-blocking activity assay employing a mouse cornea assay (Ushiro et al., FEBS Lett., 1997, Vol. 418, pp. 341-345), a commercially-available angiogenesis kit (Angiogenesis Kit, Kurabo Industries Ltd.), and a chick chorioallantoic membrane assay (Ausprunk et al., Am. J. Pathol., 1975, Vol. 97, pp. 597), besides confirming the proliferation-inhibiting action on HUVECs as carried out in Examples to be described later.

The polypeptide of the present invention can be produced through recombinant technology using a nucleic acid encoding the polypeptide. A nucleic acid to be used for recombinant production is preferably DNA, which can be obtained by determining a base sequence based on the amino acid sequence of the polypeptide of the present invention and chemically synthesizing it by the phosphoamidite method and the like or preparing it using a commercially-available DNA synthesizer. Also, the DNA can be prepared by PCR using DNA encoding PEDF as a template. The nucleic acid thus prepared, which is preferably DNA, is integrated into appropriate expression vectors, and appropriate host cells such as E. coli are transformed with the vectors thus obtained, whereby the polypeptide of the present invention can be produced through recombinant technology.

With respect to the methods for producing a polypeptide using gene recombination technology as described above, a number of various methods have been developed and used. Regarding the above, a number of various methods to prepare nucleic acids, select promoters and the like, produce recombinant vectors, determine a combination of host and vector, and transform host cells, and as far as the culture methods and the culture conditions for the transformed host cells have been developed and used. Also, a number of kits for simply carrying out the above methods have been put on the market. When the polypeptide of the present invention is produced through recombinant technology, any kind of recombination technique can be used, and specific operations may be carried out in accordance with a number of experiment operation manuals and protocols attached to kits and reagents.

Further, it is possible to express and produce the polypeptide of the present invention only in a specific site in a living body, where the polypeptide of the present invention exerts its effects, by using an expression vector capable of tissue-specific or organ-specific gene expression having a nucleic acid encoding the polypeptide of the present invention integrated thereinto. Tissue-specific or organ-specific gene expression techniques as described above are also widely known among those skilled in the art, and any of such techniques can be utilized pertaining to use of the polypeptide of the present invention.

The polypeptide of the present invention can be produced by, for example, the fluorenylmethyloxycarbonyl (Fmoc) method, the t-butyloxy carbonyl (tBoc) method, and other organic chemical synthesis methods, or by using a commercially-available appropriate peptide synthesizer. Particularly, a relatively low-molecular peptide such as P5-1 to 3 is preferably produced by organic chemical synthesis considering, for example, that mass-synthesis is possible.

Organic chemical synthesis methods of polypeptide are described by Stewart et al. (Solid Phase Peptide Synthesis, 2nd ed., 1984, Pierce Chemical Company, Rockford, I11), Bodanszky and Bodanszky (The Practice of Peptide Synthesis, 1984, Springer-Verlag, New York), and also in a number of experiment operation manuals, and the polypeptide of the present invention can be synthesized in accordance with the above methods.

Also, whether the synthesized polypeptide is as designed can be confirmed using an amino acid analyzer, high-resolution MS methods such as MALDI-TOF-MS, an amino acid sequencer, and the like. Further, the synthesized polypeptide is preferably purified before use by, for example, high-performance liquid chromatography (HPLC), as needed.

The present invention provides an anti-angiogenic pharmaceutical composition containing the aforementioned polypeptide and a pharmaceutically acceptable carrier or excipient. The present invention also provides a therapeutic agent for psoriasis containing the aforementioned polypeptide as an active ingredient, and a pharmaceutical composition for treating psoriasis containing the aforementioned polypeptide and a pharmaceutically acceptable carrier or excipient.

The pharmaceutical composition contains the aforementioned polypeptide of the present invention and one or more of pharmaceutically acceptable carriers or one or more of pharmaceutically acceptable excipients, and may further contain an additive ingredient commonly used in pharmaceutical compositions, and yet further, a medicinal ingredient other than the polypeptide of the present invention. The pharmaceutical composition as described above can be prepared as an oral formulation, an external agent, a percutaneous-absorption formulation, a transmucosal formulation, an eye drop, an injection, and various kinds of other formulations suitable for intended administration route. Further, the pharmaceutical composition of the present invention can also be prepared in the form of, for example, solid agent, liquid agent, suspension, emulsified agent, ointment, liposome agent, and sustained-release agent. Preparation of the pharmaceutical composition as described above can be carried out by drug preparation techniques publicly or commonly known by those skilled in the art.

It is preferable to prepare the pharmaceutical composition of the present invention in the form of external agent. The external agent can be prepared in the form of, for example, ointment, liquid, lotion, cream, patch, spray, and emulsified formulation. The polypeptide of the present invention has a size of 120 amino acid residues or less and thus is more highly absorbable through the skin barrier compared to general protein; therefore, it is suitable to be used as an external agent.

Dosage of the pharmaceutical composition of the present invention is appropriately set according to physician's judgment based on the state of the disease requiring administration of the pharmaceutical composition, age, and other medical factors. However, the amount of the polypeptide of the present invention to be added to the pharmaceutical composition is adjusted to be roughly within a range of 0.1 to 99% (w/w) per weight of the composition, and then the dosage of the pharmaceutical composition may be controlled, as needed.

As described in Examples to be explained below, the polypeptide of the present invention has a physiological activity equivalent to PEDF, and thus is applicable to various diseases for which PEDF has been approved. Particularly, the polypeptide of the present invention has an anti-angiogenic action, and thus is effective in the treatment of diseases involving undesirable angiogenesis. The diseases involving undesirable angiogenesis that can be treated with the polypeptide of the present invention include, but are not limited to, malignant neoplasm (tumor), malignant melanoma, arteriosclerosis, chronic rheumatoid arthritis, diabetic retinopathy, angiogenesis-associated age-related macular degeneration, and psoriasis vulgaris.

Particularly, the polypeptide of the present invention is effective in the treatment of psoriasis vulgaris. Psoriasis is a skin disease involving angiogenesis of capillary vessels in the dermis with a clinical characteristic of appearance of erythematous dry plaques covered by grayish white or silver white scales mainly on the skin. Particularly, skin eruption in the exposed part greatly reduces the patient's QOL, and further, it is a disease that may also develop fever with joint symptoms and pustule.

Pharmacotherapeutics of psoriasis include topical administration of, for example, emollient, salicylic acid, steroid external agent, vitamin D3 external agent (calcipotriol), etretinate, or cyclosporine. Also, photochemotherapy involving administration of psoralen and long-wavelength ultraviolet radiation is occasionally employed. However, the above treatments are not necessarily satisfactory in terms of therapeutic effects, and many of them are associated with side effects such as skin irritation. Particularly, in terms of side effects, a steroid external agent causes skin atrophy and the like, a vitamin D3 external agent causes hypercalcaemia and the like, and cyclosporine causes renal disorders and the like.

The polypeptide of the present invention or the pharmaceutical composition containing the polypeptide has a polypeptide derived from human protein as an active ingredient and exhibits an excellent effect, and also it is considered as a medicine having advantages such that it causes almost no side effects. Based on the foregoing, the polypeptide of the present invention or the pharmaceutical composition containing the polypeptide provides novel pharmacotherapeutics for psoriasis vulgaris.

Hereinbelow, the present invention will be described further in detail with Examples. However, the below-described Examples are provided for illustrative purposes only and they do not limit the present invention in any way. Further, unless otherwise specifically noted, experiment operations in Examples were carried out in accordance with descriptions by Maniatis T. et al. (Molecular Cloning, a Laboratory Manual, Cold Spring harbor Laboratory, New York, 1982) and other experiment operation manuals, or descriptions of instructions attached to commercially available reagents or kits.

### Examples

### Example 1

### 1) Preparation of cDNA encoding PEDF

In accordance with the description by Yamagishi et al. (Biochem. Biophys. Res. Commun., 2002, Vol. 296, pages 877-882), PCR was carried out using a pair of primer DNAs shown below with respect to a human placenta cDNA library (Clontech Laboratories, Inc.)
Primer 1F: 5'-CTCAGTGTGCAGGCTTAGAG-3' (SEQ ID NO: 7)
Primer 1R: 5'-CCTTCGTGTCCTGTGGAATC-3' (SEQ ID NO: 8)

Amplification products of approximately 1300 bp were collected from an agarose gel and linked to the SmaI site of pBluescriptIIKS. The construct thus obtained was confirmed by restriction enzyme digestion and sequencing. The above vector was cleaved with XbaI and HindIII and fragments were collected, which were then cloned between the NheI site (blunt end) and the XbaI site of pBK-CMV (Stratagene Corporation), whereby pBK-CMV-PEDF plasmids, which were expression vectors having cDNA encoding PEDF, were produced.

### 2) Production of polypeptides F1 to F3

Using the vectors produced in 1) as templates, PCR was carried out employing three pairs of primers shown below.
Primer 2F: 5'-AAACATATGCAGGCCCTGGTGCTACTCCTCTGCAT-3' (SEQ ID NO: 9)
Primer 2R: 5'-CCCGTCGACTTATGACTTTTCCAGAGGTGCCACAAA-3' (SEQ ID NO: 10)
Primer 3F: 5'-AAACATATGTATGGGACCAGGCCCAGAGTCCTGA-3' (SEQ ID NO: 11)
Primer 3R: 5'-CCCGTCGACTTAGTCATGAATGAACTCGGAGGTGA-3' (SEQ ID NO: 12)
Primer 4F: 5'-GGGCATATGATAGACCGAGAACTGAAGACCGTGCA-3' (SEQ ID NO: 13)
Primer 4R: 5'-AAAGTCGACTTAGGGGCCCCTGGGGTCCAGAAT-3' (SEQ ID NO: 14)

DNA fragments amplified by the primers 2F and 2R encode a polypeptide comprising the amino acid sequence of positions 1 to 160 of PEDF (F1), DNA fragments amplified by the primers 3F and 3R encode a polypeptide comprising the amino acid sequence of positions 161 to 300 of PEDF (F2), and DNA fragments amplified by the primers 4F and 4R encode a polypeptide comprising the amino acid sequence of positions 301 to 418 of PEDF (F3), respectively.

Each of the amplification products obtained by the aforementioned PCR reaction was purified and then cleaved with NdeI and SalI. The products thus obtained were then linked to the multicloning site of pGEX-6P-1 (Amersham Biosciences Corp.) that had been ring-opened similarly with NdeI and SalI, whereby vectors pGEX-6P-F1 to -F3 expressing each of F1 to F3 were obtained. Further, from E. coli transformed with each of the vectors (JM109), polypeptides F1 to F3 were produced in accordance with the method described by Walker et al. (Biotechnology, 1994, Vol. 12, pages 601 to 605).

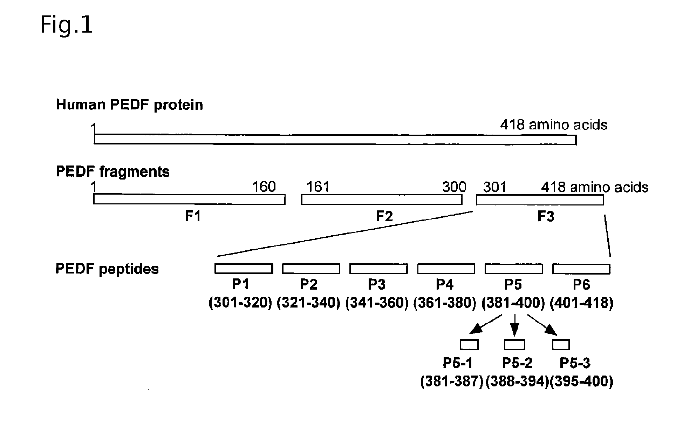

Furthermore, polypeptides P1 to P6 comprising the amino acid sequences obtained by segmenting the amino acid sequence of positions 301 to 418 of PEDF by 20 amino acid residues (only P6 would have 16 amino acid residues) were chemically synthesized by the Fmoc solid-phase synthesis using a peptide synthesizer (Sigma-Aldrich Corporation). Further, polypeptides P5-1 to P5-3 comprising each of the amino acid sequences of positions 381 to 387, 388 to 393, and 394 to 400 of PEDF were chemically synthesized using a peptide synthesizer. Figure 1 shows a schematic diagram of the arrangement of each polypeptide with respect to PEDF.

### 3) Measurement of activity

The inhibitory effect of each polypeptide on tumor cell proliferation with respect to human osteosarcoma cells, MG63 (Health Science Research Resources) was measured in accordance with the method described by Takenaka et al. (Life Science, 2005, Vol. 77, pages 3231-3241).

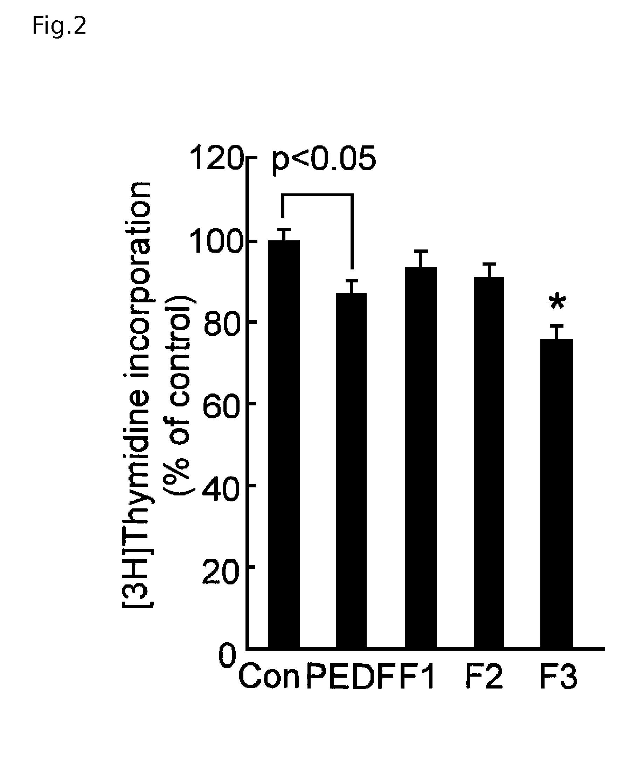

Into Dulbecco's modified Eagle's medium (DMEM) supplemented with 10% of fetal bovine serum (FBS) and 100 units/mL penicillin and streptomycin (hereinbelow, simply referred to as a medium), MG63 was suspended. Then, 100 nM commercially available PEDF protein (Sigma) and each of 100 nM polypeptides F1 to F3 prepared in Example 1 "2) Production of polypeptides F1 to F3" were each added per medium, followed by incubation at 37°C for 24 hours. Subsequently, [³H] thymidine (Amersham Biosciences Corp.) was added and incubation was carried out for four hours, after which the amount of [³H] thymidine incorporation by MG63 was measured and the inhibitory effect on tumor cell proliferation was examined. The results thus obtained are shown in Figure 2.

As shown in Figure 2, compared to a control to which PEDF protein was added, a strong inhibitory effect on tumor cell proliferation was confirmed with polypeptide F3 (Figure 2, p < 0.01).

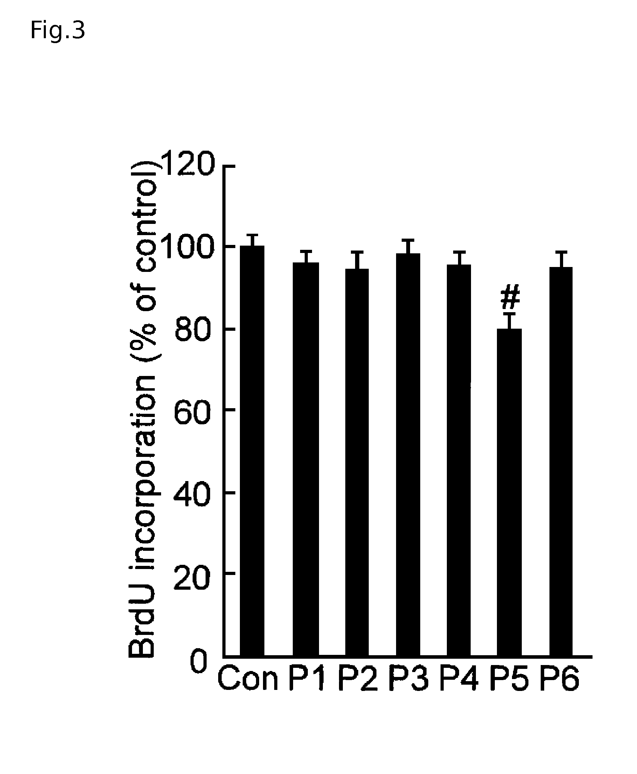

Also, by carrying out similar operations to the above, the inhibitory effect on MG63 tumor cell proliferation was examined by measuring the amount of 5-bromo-2'deoxyuridine (Roche) incorporation with addition of each of 100 nM polypeptides P1 to P6. The results thus obtained are shown in Figure 3.

As shown in Figure 3, an inhibitory effect on tumor cell proliferation was confirmed with polypeptide P5 (Figure 3, P < 0.01).

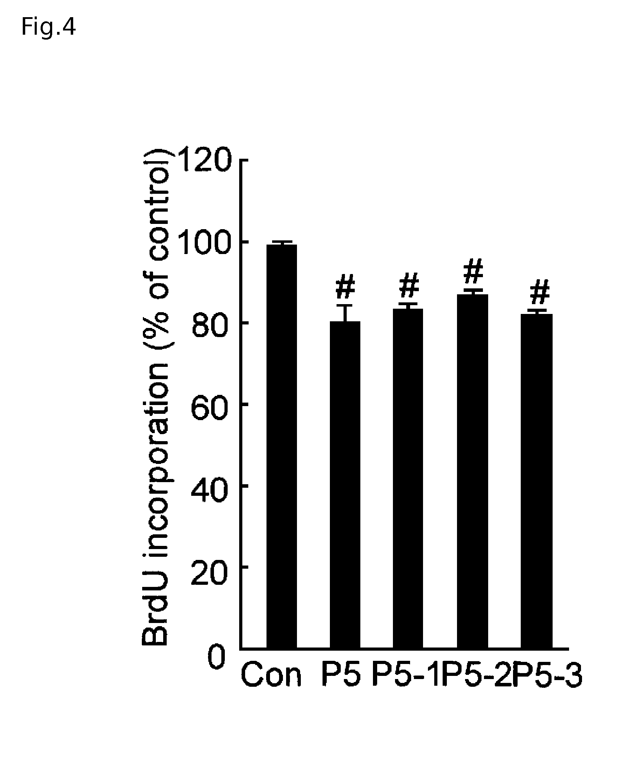

Further, by carrying out similar operations to the above, the inhibitory effect on MG63 tumor cell proliferation was examined by measuring the amount of 5-bromo-2'deoxyuridine incorporation with addition of each of 100 nM polypeptides P5-1 to P5-3. The results thus obtained are shown in Figure 4.

As shown in Figure 4, an inhibitory effect on tumor cell proliferation similar to polypeptide P5 was confirmed with any of polypeptides P5-1 to P5-3 (Figure 4, p < 0.01).

### 4) Inhibitory effect on proliferation of normal human umbilical vein endothelial cells (HUVECs)

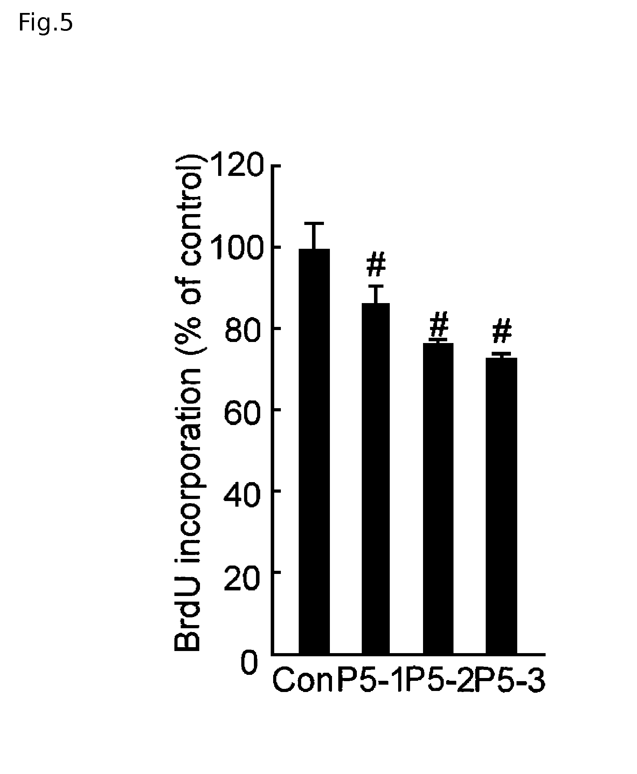

To each well of a 96-well plate, HUVECs were seeded at 10³ cells/100 µL, and 100 nM commercially available PEDF protein comprising the amino acid sequence of SEQ ID NO: 6 (Sigma), 100 ng/mL VEGF (R&D systems, Inc.), and each of 100 nM polypeptides P5-1 to P5-3 were each added to the wells, followed by incubation for two to four days under conditions of 37°C and 5% CO₂**.** Subsequently, the amount of 5-bromo-2'deoxyuridine incorporation by HUVECs was measured. The results thus obtained are shown in Figure 5.

As shown in Figure 5, any of polypeptides P5-1 to P5-3 significantly inhibited proliferation of HUVECs in comparison with a control to which PEDF protein was added (Figure 5, p < 0.01).

### 5) External therapeutic effect on the skin

The hair in the area of approximately 1 cm² in the back of 7 to 8 week-old SCID mice (Clea Japan, Inc.) was shaved, and the full-thickness skin was removed while keeping the vessel plexus intact on the fascia overlying back muscles. The skin of affected area of a psoriasis patient that was sampled under the approval of the ethical code was washed with PBS containing 1% penicillin, 1% streptomycine, and 1% amphotericin B, and each piece of the skin thus treated was separately transplanted onto the skin-removed area of the above-described mice.

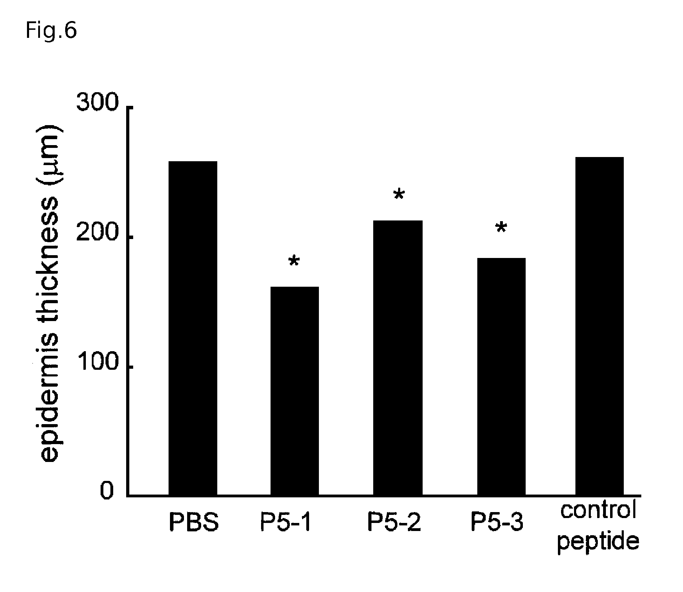

Seven days after transplantation, 70 µL of each of solutions prepared by dissolving polypeptides P5-1, P5-2, and P5-3 into phosphate buffered saline (PBS) (each 1 mM) was applied to the grafted sites of three mice daily for 14 days. As a result, no side effects and the like were apparent at the applied sites. Further, as a control,
the same volumes of PBS solution containing a polypeptide having a random sequence and PBS without polypeptide were applied. After 14 days, a part of the grafted skin was collected and embedded in an optimal cutting temperature (OCT) reagent (Sakura finetechnical Co., Ltd.) and then frozen in liquid nitrogen, which was then prepared as 5 µm-thick sections. The above-described application test was repeated three times, and the results thus obtained were shown in Figure 6.

As shown in Figure 6, based on the observation of sections stained with hematoxylin-eosin (HE), the thickness of the grafted skin administered with any of polypeptides P5-1, P5-2, and P5-3 was significantly reduced (Figure 6, p < 0.05).

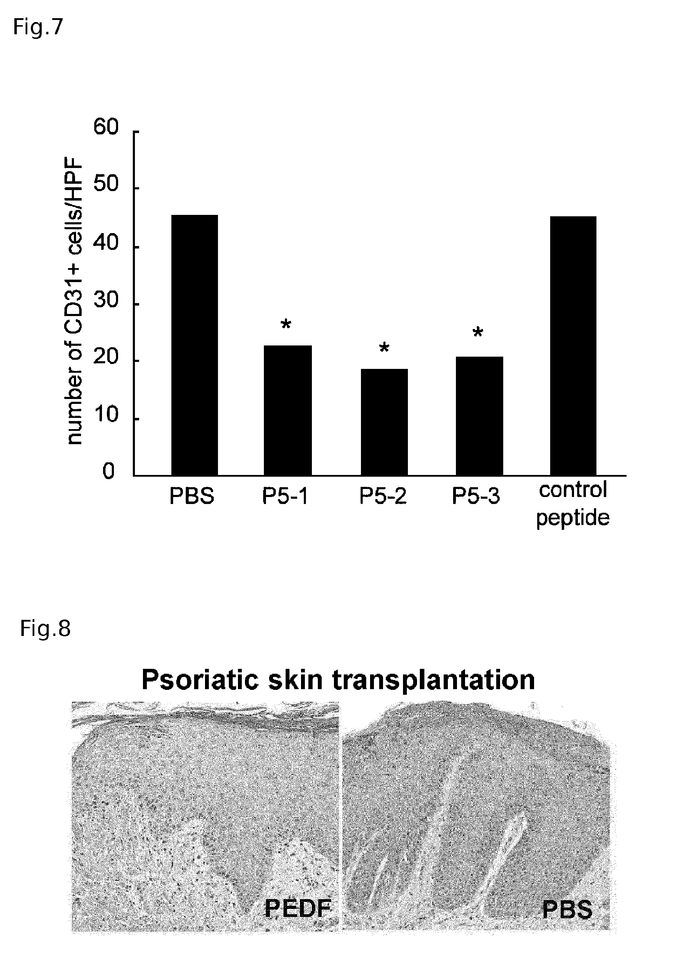

Also, in accordance with the method of Abe et al. (Am. J. Pathol., 2004, Vol. 164, No. 4, pages 1225-1232), the capillary endothelial cells in the grafted superficial dermis were enumerated by immunoassay employing a rat anti-mouse CD31 antibody using CD31, which was a marker protein of the capillary endothelial cells, as an index. The results thus obtained are shown in Figure 7.

As shown in Figure 7, the number of capillary endothelial cells significantly decreased (Figure 7).

Further, in a similar manner to the above, the hair in the area of approximately 1 cm² in the back of 7 to 8 week old SCID mice (Clea Japan, Inc.) was shaved, and the full-thickness skin was removed while keeping the vessel plexus intact on the fascia overlying back muscles. The skin of affected area of a psoriasis patient and the skin of a normal individual that were sampled under the approval of the ethical code were each washed with PBS containing 1% penicillin and 1% amphotericin B, and each of the skin thus treated was separately transplanted onto the skin-removed area of the above-described mice.

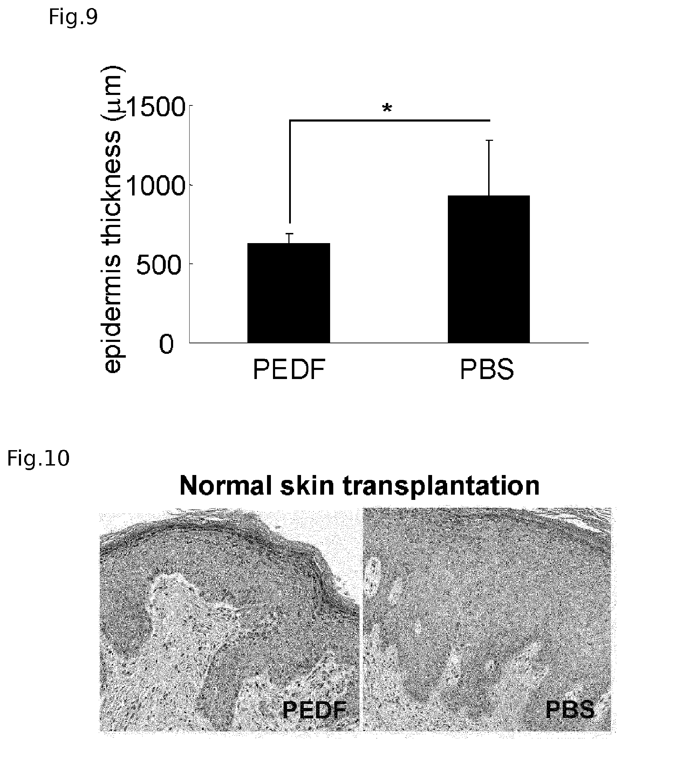

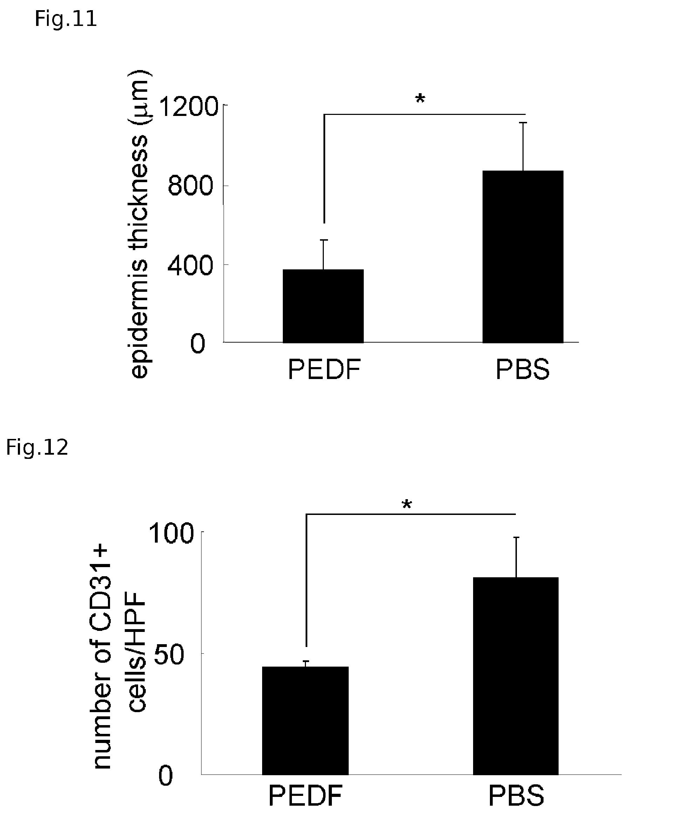

Seven days after transplantation, commercially available PEDF protein (30 µg) was given at the grafted site by subcutaneous injection. The injection was administered for three weeks at three times/week. As a control, a mouse given subcutaneous injection of only PBS was used. The day after the last subcutaneous injection, a part of the grafted skin was collected and stained with HE in a similar manner to the above, and the thickness of the grafted skin was measured. The results obtained from the skin of affected area of a psoriasis patient are shown in Figure 8 (photograph) and Figure 9, and the results obtained from the skin of a normal individual are shown in Figure 10 (photograph) and Figure 11.

As shown in Figures 8, 9, 10, and 11, thickness of the skin of a normal individual and the skin of affected area of a psoriasis patient was both significantly reduced (Figures 9 and 11, both with p < 0.05).

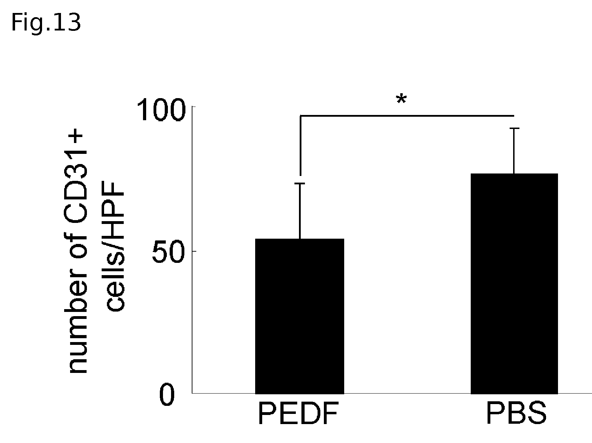

Also, by carrying out similar operations to the above, the capillary endothelial cells were enumerated using CD31 as an index. The results obtained from the skin of affected area of a psoriasis patient are shown in Figure 12 and the results obtained from the skin of a normal individual are shown in Figure 13.

As shown in Figures 12 and 13, the number of capillary endothelial cells was significantly reduced in both of the skin of a normal individual and the skin of affected area of a psoriasis patient in a group administered with PEDF protein (Figures 12 and 13, both with p < 0.05).

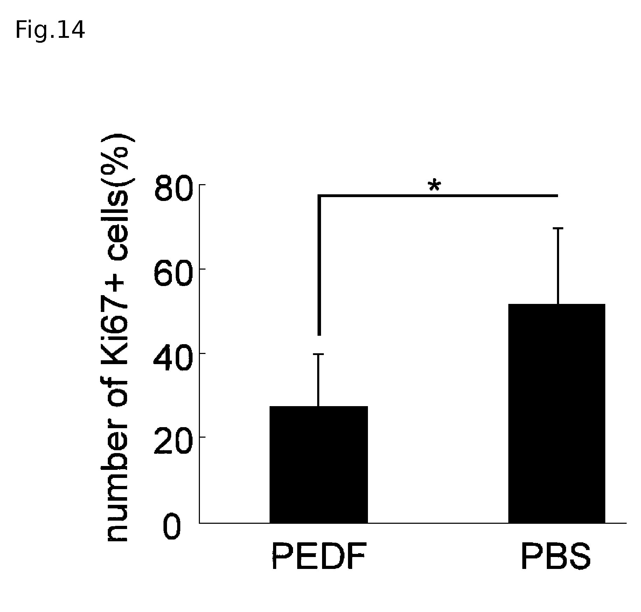

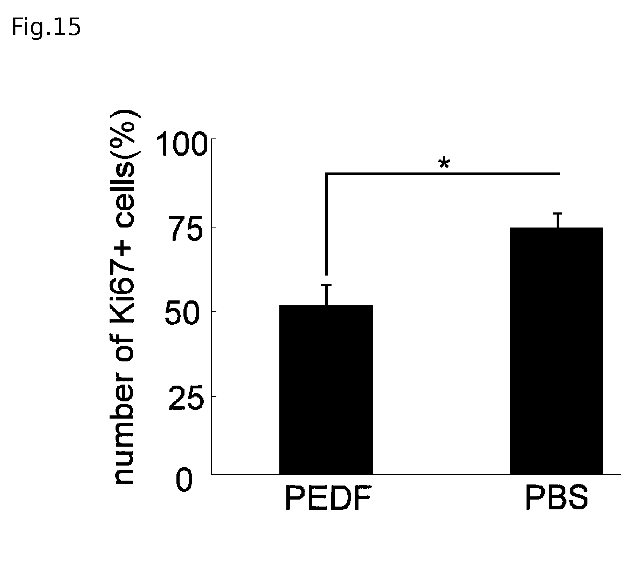

Further, the frequency of Ki-67 expressing positive cells, where Ki-67 is a proliferation marker of the basal cell layer, was measured in accordance with the method of Gilhar et al. (Am. J. Pathol., 2006, Vol. 168, No. 1, pages 170 to 175). The results obtained from the skin of affected area of a psoriasis patient are shown in Figure 14 and the results obtained from the skin of a normal individual are shown in Figure 15.

As shown in Figures 14 and 15, the frequency was significantly reduced in both of the skin of a normal individual and the skin of affected area of a psoriasis patient in a group administered with PEDF protein, suggesting that proliferation of the basal cells was inhibited (Figures 14 and 15, both with p < 0.05).

## Claims

1. A polypeptide having an anti-angiogenic action, comprising any one or more of amino acid sequences shown in SEQ ID NOs: 1 to 3 and having 120 or less amino acid residues.

2. The polypeptide according to claim 1, comprising the amino acid sequence shown in SEQ ID NO: 4.

3. The polypeptide according to claim 1, having 20 or less amino acid residues.

4. The polypeptide according to claim 3, comprising the amino acid sequence shown in SEQ ID NO: 5.

5. The polypeptide according to claim 1, consisting of any of the amino acid sequences shown in SEQ ID NOs: 1 to 3.

6. A therapeutic agent for psoriasis comprising a polypeptide comprising the amino acid sequence shown in SEQ ID NO: 6 and having an anti-angiogenic action as an active ingredient.

7. An anti-angiogenic agent comprising the polypeptide according to any of claims 1 to 5 as an active ingredient.

8. A therapeutic agent for psoriasis comprising the polypeptide according to any of claims 1 to 5 as an active ingredient.

9. A tumor cell proliferation-inhibiting agent comprising the polypeptide according to any of claims 1 to 5 as an active ingredient.

10. An anti-angiogenic pharmaceutical composition comprising the polypeptide according to any of claims 1 to 5 and a pharmaceutically acceptable carrier or excipient.

11. A pharmaceutical composition for treating psoriasis comprising the polypeptide according to any of claims 1 to 5 and a pharmaceutically acceptable carrier or excipient.

12. The pharmaceutical composition according to claim 10 or 11, being provided as an external agent.
